# EUROPÄISCHE PATENTSCHRIFT

(11) **EP 4 014 926 B1**
(45) Veröffentlichungstag und Bekanntmachung des Hinweises auf die Patenterteilung: **21.05.2025**
(21) Anmeldenummer: 20214675.9
(22) Anmeldetag: 16.12.2020
(51) Int. Cl.: A61C 19/04, A61C 5/80

(54) **DENTALOBJEKT ZUM BEFESTIGEN AN EINEM ZAHN**
DENTAL OBJECT FOR FIXING TO A TOOTH
OBJET DENTAIRE DESTINÉ À ÊTRE FIXÉ SUR UNE DENT

(43) Veröffentlichungstag der Anmeldung: 22.06.2022
(73) Patentinhaber: Ivoclar Vivadent AG, 9494 Schaan (LI)
(72) Erfinder: TICHY, Raimund, 6800 Feldkirch (AT); REINHARDT, Jonas, 7206 Igis (CH)
(74) Vertreter: Baldus, Oliver

(56) Entgegenhaltungen:
- WO-A1-03/096922
- US-A1- 2008 026 344
- US-A1- 2011 123 959
- US-A1- 2020 129 269

## Beschreibung

Die vorliegende Erfindung betrifft ein Dentalobjekt zum Befestigen an einem Zahn oder an Zähnen.

Heutige intraorale Befestigungsmöglichkeiten sind durch mechanische Klemmen oder chemisch durch Verkleben (Adhäsion) realisiert. Bekannte Klemmverfahren umfassen beispielsweise Klemmen, die um einen oder mehrere Zähne gelegt werden, dentale Aligner, die den ganzen oder Teile eines Zahnbogens umfassen, oder molare Bänder, die um einen oder mehrere Zähne gelegt werden.

Eine Adhäsion von Fremdkörpern wird mittels Klebemittel oder Zementen erreicht. Eine weitere reversible Möglichkeit der Befestigung ist das Ansaugen mittels Haftcreme. Dies funktioniert aber nur auf dem Zahnfleisch, nicht aber auf dem Zahn selbst. Die Haftcreme funktioniert zudem nur auf größeren Flächen. Weiter bieten moderne Haftcremes nur schwache Befestigung, so dass das Tragen eines verschluckbaren Fremdkörpers über Nacht unmöglich ist.

Die Druckschrift US2011/123959 A1 betrifft ein Zahnkern- und - Pfostensystem zur Wiederherstellung von Zähnen mit einer Kernvorrichtung und einem Zahnpfosten, der als ein stabähnliches Element ausgebildet ist.

Die Druckschrift US 2008/026344 A1 betrifft einen künstlichen Zahn, der aus oberen und unteren Teilen aufgebaut ist, die räumlich zueinander einstellbar sind.

Die Druckschrift WO 03/096922 A1 betrifft ein kieferorthopädisches Bracket zur Verankerung einer festsitzenden kieferorthopädischen Apparatur an einem Zahn, ein mit einer an einem Zahn zu verankernden Bracketbasis und einer Bracketbefestigung zur Befestigung einer Kraft-Druck- und/oder Drehmomentaufbringungseinrichtung.

Es ist daher die technische Aufgabe der vorliegenden Erfindung, ein Dentalobjekt fest und doch abnehmbar an einem Zahn oder an Zähnen zu befestigen.

Diese Aufgabe wird durch den Gegenstand nach dem unabhängigen Anspruch gelöst. Technisch vorteilhafte Ausführungsformen sind Gegenstand der abhängigen Ansprüche, der Beschreibung und der Zeichnungen.

Gemäß einem ersten Aspekt wird die technische Aufgabe durch ein Dentalobjekt zum Befestigen an einem Zahn oder Zähnen in einem Intraoralraum gelöst, mit einem Haftbereich, der Gutta-Percha zum Befestigen des Dentalobjekts an dem Zahn oder Zähnen umfasst. Dadurch wird der technische Vorteil erreicht, dass der Patient in der Lage ist, das Dentalobjekt jederzeit zu entfernen und wiedereinzusetzen. Durch die Entfernbarkeit kann eine Plaquebildung unter dem oder um das Dentalobjekt nach einem Entfernen gereinigt werden. Das Dentalobjekt kann lingual, labial, buccal oder an anderen Stellen am Zahn befestigt werden. Die Zähne werden durch das Dentalobjekt nicht geschädigt, da keine Ätzung, keine Klemmen, keine Bügel und kein Metall verwendet werden. Die Zähne werden nicht verschoben, wie beispielsweise bei einem molaren Band und es wird keine Haftcreme benötigt.

Erfindungsgemäß umfasst das Dentalobjekt eine Sensorik, ein Elektronikbauteil, eine Batterie, ein Gehäuse und/oder eine Halteplatte. Das Gehäuse kann beispielsweise aus Kunststoff oder Metall gebildet sein. Dadurch wird beispielsweise der technische Vorteil erreicht, dass weitere Komponenten in dem Gehäuse angeordnet werden können.

In einer weiteren technisch vorteilhaften Ausführungsform des Dentalobjekts ist eine Schicht des Gutta-Percha-Materials an dem Gehäuse befestigt. Dadurch wird beispielsweise der technische Vorteil erreicht, dass das Gehäuse direkt am Zahn befestigt werden kann.

In einer weiteren technisch vorteilhaften Ausführungsform des Dentalobjekts ist die Schicht des Gutta-Percha-Materials mittels einer Klebeschicht an dem Gehäuse befestigt und/oder das Gehäuse umfasst eine strukturierte Oberfläche zum Befestigen des Gutta-Percha-Materials und/oder ein oder mehrere Rastelemente zum Befestigen des Gutta-Percha-Materials und/oder ein oder mehrere Verbindungselemente zum Herstellen eines Formschlusses zwischen einem Haftbereich und dem Sensor oder Sensorgehäuse. Die Klebeschicht und die anderen Verbindungs- und Rastelemente bewirkt eine starke Verbindung zwischen dem Gehäuse und dem Gutta-Percha-Material. Das Gehäuse kann auch eine strukturierte Kontaktfläche oder Öffnungen aufweisen, in die das Gutta-Percha-Material eingelassen ist. Dadurch wird beispielsweise der technische Vorteil erreicht, dass eine Ablösung des Gutta-Percha-Materials von dem Gehäuse verhindert wird.

In einer weiteren technisch vorteilhaften Ausführungsform des Dentalobjekts weist die Schicht des Gutta-Percha-Materials eine Dicke von 1 µm bis 5 mm auf. Dadurch wird beispielsweise der technische Vorteil erreicht, dass eine gute Haftung des Dentalobjektes erreicht wird und eine individuelle Anpassung für jeden Patienten möglich ist.

In einer weiteren technisch vorteilhaften Ausführungsform des Dentalobjekts umfasst das Gutta-Percha-Material eine vorgefertigte Durchgangsöffnung zum Führen von Flüssigkeit vom Zahn in das Innere des Gehäuses. Dadurch wird beispielsweise der technische Vorteil erreicht, dass Speichel im Inneren des Gehäuses durch einen Sensor analysiert werden kann.

In einer weiteren technisch vorteilhaften Ausführungsform des Dentalobjekts umfasst das Gutta-Percha-Material antibakterielle Partikel oder Substanzen. Dadurch wird beispielsweise der technische Vorteil erreicht, dass bakterielle Infektionen beim Tragen des Dentalobjekts verhindert werden können. Das Gutta-Percha-Material kann auch Substanzen umfassen, die den pH-Wert lokal regulieren, so dass keine Karies entstehen kann.

In einer weiteren technisch vorteilhaften Ausführungsform des Dentalobjekts umfasst das Gutta-Percha-Material einen Weichmacher. Der Weichmacher kann beispielsweise mittelkettige Triglyceride (Miglyol), Glycerin oder andere Fette, wie beispielsweise Olivenöl umfassen. Dadurch wird beispielsweise der technische Vorteil erreicht, dass sich der Haftbereich besser an den Zahn anpassen kann.

In einer weiteren technisch vorteilhaften Ausführungsform des Dentalobjekts umfasst das Gutta-Percha-Material, der Sensor und/oder das Gehäuse ein oder mehrere Kanäle zum Führen von Speichel zu einer Sensoreinheit. Die Kanäle sind beispielsweise durch Vertiefungen gebildet. Die Kanäle können zum Durchleiten von Flüssigkeit vorgesehen sein. Dadurch wird beispielsweise der technische Vorteil erreicht, dass Flüssigkeitsdurchlauf im Kontaktbereich gefördert wird.

In einer weiteren technisch vorteilhaften Ausführungsform des Dentalobjekts erstrecken sich die Kanäle schräg oder vertikal durch das Gutta-Percha-Material, den Sensor und/oder das Gehäuse. Dadurch wird beispielsweise der technische Vorteil erreicht, dass ein Speichelfluss hin zu einer Sensoreinheit erzeugt wird.

In einer weiteren technisch vorteilhaften Ausführungsform des Dentalobjekts ist das Gutta-Percha-Material anatomisch vorgeformt. Dadurch wird beispielsweise der technische Vorteil erreicht, dass eine schnellere Anpassung des Haftbereichs stattfindet.

Das Dentalobjekt umfasst beispielsweise eine Elektronik und einen Sender, wie beispielsweise NFC-Schaltung, oder Bluetooth. Dadurch wird beispielsweise der technische Vorteil erreicht, dass Daten durch das Dentalobjekt gewonnen und verarbeitet werden können. Ein Verfahren zum Befestigen eines Dentalobjekt an einem Zahn oder an Zähnen umfasst den Schritt eines Aufdrückens eines Haftbereichs des Dentalobjekts, der Gutta-Percha-Material umfasst, auf den Zahn. Durch das Verfahren werden die gleichen technischen Vorteile wie durch das Dentalobjekt nach dem ersten Aspekt erreicht.

In einer technisch vorteilhaften Ausführungsform des Verfahrens wird das Gutta-Percha-Material vor dem Aufdrücken des Haftbereichs erwärmt. Dadurch wird beispielsweise der technische Vorteil erreicht, dass sich der Haftbereich besser an den Zahn anpasst und eine starke Haftwirkung erreicht wird. Eine Verwendung von Gutta-Percha dient zum Befestigen eines Dentalobjekts an einem Zahn oder an Zähnen. Durch die Verwendung werden die gleichen technischen Vorteile wie durch das Dentalobjekt nach dem ersten Aspekt erreicht.

Ausführungsbeispiele der Erfindung sind in den Zeichnungen dargestellt und werden im Folgenden näher beschrieben.

Es zeigen:
- Fig. 1: eine schematische Ansicht eines Dentalobjektes.
- Fig. 2: eine weitere schematische Ansicht eines Dentalobjekts; und
- Fig. 3: eine schematische Ansicht eines plastischen Haftbereichs.

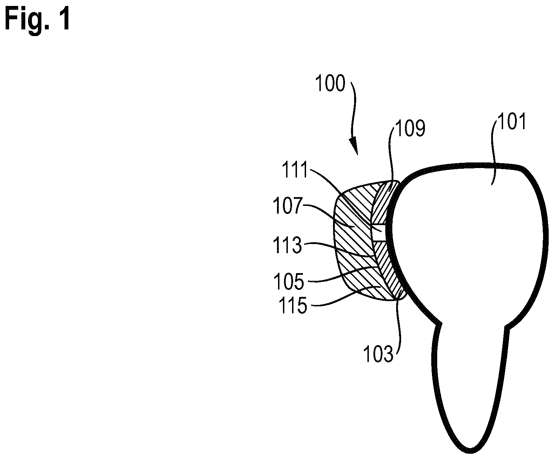

Fig. 1 zeigt eine schematische Ansicht eines Dentalobjektes 100 zur Befestigung an einem Zahn 101. Das Dentalobjekt 100 weist einen Haftbereich 103 auf, der Gitta-Percha-Material 105 zum Befestigen des Dentalobjekts 100 an dem Zahn 101 umfasst. Zum Befestigen wird der plastische Haftbereich 103 auf den Zahn 101 gedrückt. Das Gutta-Percha-Material 105 kann hierzu beispielsweise mit einer Heißluftquelle oder im Wasserbad erhitzt werden und im erhitzten Zustand (~ 50 °C) in den Mund eines Patienten eingesetzt werden. Das Gutta-Percha-Material 105 verbindet sich während dem Abkühlen auf Mundtemperatur mit dem Zahn 101 und dem Zahnfleisch, so dass das Dentalobjekt 100 im Mund abgeformt und fixiert wird. Das Gutta-Percha-Material 105 erzeugt eine haftende Wirkung am Zahn 101.

Das Gutta-Percha-Material 105 ist ein plastischer, gummiartiger, kautschukähnlicher Stoff aus dem eingetrockneten, koagulierten Milchsaft von Sapotengewächsen (Sapotaceae) (Payena spp. und Palaquium spp.) sowie von Spindelsträuchern (Euonymus spp.) oder dem Guttaperchabaum (Palaquium gutta). Das Gutta-Percha-Material 105 umfasst Gutta-Percha. Gutta-Percha weist folgende Molekülstruktur auf, bei der die Anzahl n der Wiederholeinheiten typischerweise im Bereich von 100 liegt. Das Gutta-Percha ist unvernetzt. Gutta-Percha weist eine hohe Biokompatibilität, Dimensionsstabilität und plastische Verformbarkeit auf.

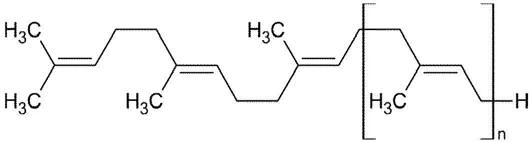

Durch das Dentalobjekt 100 mit dem Haftbereich 103 aus Gutta-Percha-Material 105 wird der technische Vorteil erreicht, dass nicht-prothetische und nicht-orthodontische Dentalobjekte 100 im Intraoralbereich entfernbar befestigt werden können.

Das Dentalobjekt 100 kann folgende Bestandteile umfassen: Sensoren, Elektronikbauteile, Batterien, oder Gehäuse für verschiedene Bestandteile. Das Gehäuse 107 kann aus einem Kunststoff, Karbon oder Metall gefertigt sein. Das Dentalobjekt 100 kann ein Objekt sein, das bereits eine anatomisch auf den Zahn 101 angepasste Form aufweist. Ein derartiges Objekt kann beispielsweise mittels eines 3D-Druckers individuell erzeugt und an die spezielle Form eine Zahns 101 eines Patienten angepasst sein, wie beispielsweise ein individuell geformter Zahnaufsatz. In Allgemeinen kann das Dentalobjekt 100 jedes Objekt sein, das im Mundraum an dem Zahn 101 befestigt werden soll.

Das Gutta-Percha-Material 105 kann in einer Schicht 109 als Haftbereich 103 an dem Dentalobjekt 100 angeordnet sein. Die Schicht 109 weist beispielsweise eine Dicke von 1 µm bis 5 mm auf. Das Dentalobjekt 100 und/oder der Haftbereich 103 können anatomisch vorgeformt sein, indem diese eine konkave Einwölbung 113 umfassen, in der der Zahn 101 liegt. Es können auch mehrere Einwölbungen 113 vorgesehen Sein, in denen dann jeweils ein Zahn 101 liegt.

Dadurch liegt das Dentalobjekt 100 besser am Zahn 101 an. In der Schicht 109 kann eine vorgefertigte Durchgangsöffnung 111 zum Führen von Flüssigkeit vom Zahn in das Innere des Gehäuses 107 vorgesehen sein. Dadurch kann die Flüssigkeit von dem Zahn 101 im Inneren des Gehäuses 107 von einem Sensor analysiert werden.

Das Gutta-Percha-Material 105 kann antibakterielle Partikel umfassen, wie beispielsweise durch einen Einbau von Silberpartikeln, Kupferpartikeln oder eine Mischung mit Chlorhexidin and Chloroxylenol. Zudem kann das plastische Material Antibiotika umfassen, wie beispielsweise Penicillin, Clindamycin, Erythromycin, Cefadroxil, Metronidazol und/oder Tetracycline. Zudem können pH-regulierende Füllmaterialen verwendet werden, wie Kalziumfluorid, Kalziumhydroxid oder alkalische Glasfüller, wie Kalzium-Fluorosilicat-Glas oder ionenfreisetzende Materialien, wie beispielsweise von F-, OH-, oder Ca2+.

Bei individuellen Dentalobjekten 100, die räumlich und anatomisch an den Zahn 101 angepasst sind, kann die Dicke der Schicht 109 gering sein und in einem Bereich von 200 µm liegen. Bei vorgefertigten, standardmäßigen Dentalobjekten 100 liegt die Dicke der Schicht 109 im Bereich von Millimetern, da eine größere plastische Verformung der Schicht 109 stattfindet. Die Abmessungen der Schicht 109 in Länge und Breite können beispielsweise 10 mm mal 10 mm betragen. Im Allgemeinen können jedoch auch andere Abmessungen gewählt werden, sofern diese zweckdienlich sind.

Aufgrund der Befestigung mit dem Gutta-Percha-Material 105 kann das Dentalobjekt 100 jederzeit entfernt werden und nach einem Erhitzen wiedereingesetzt werden. Dabei stellt auch eine feuchte Umgebung (Speichel) kein Problem dar, da sich das Gutta-Percha-Material 105 nicht in Wasser auflöst. Zudem ist Gutta-Percha biokompatibel.

Die Befestigung des Dentalobjekts 100 mit dem Gutta-Percha-Material ist stärker als mit anderen üblichen Materialien. Dadurch kann eine reversible Befestigung des Dentalobjekts 100 im Intraoralraum erreicht werden. Das Gutta-Percha-Material 105 wird hierbei als Haftvermittler verwendet. Der Vorteil des Gutta-Percha-Materials 105 ist, dass dieses auch auf gekrümmten Flächen eingesetzt werden kann. Die Verbindung kann nach einem kurzen Aufheizen vor dem Wiedereinsetzen mehrmals verwendet werden. Die Verbindung ist zahnschonend, da kein Ätzen der Zahnsubstanz erforderlich ist. Zum Erleichtern des Ablösens kann erneut ein Erwärmen des Gutta-Percha-Materials durchgeführt werden.

Ein Erhitzen oder Erwärmen des Gutta-Percha-Materials 105 vor dem Aufdrücken des Dentalobjekts 100 kann mittels eines Heißluftgebläses oder eines Wasserbades erfolgen. Das Dentalobjekt 100 kann jedoch auch eine elektrische oder chemische Erwärmungsvorrichtung umfassen, die von einem Benutzer beim Einsetzen des Dentalobjekts 100 aktiviert wird. Die elektrische Erwärmungsvorrichtung kann beispielsweise durch eine Heizwendel gebildet sein. Die chemische Erwärmungsvorrichtung kann durch zwei Substanzen gebildet sein, die miteinander in Reaktion gebracht werden und dabei die Wärme für das Gutta-Percha-Material 105 erzeugen. Durch die Erwärmungsvorrichtung wird das Gutta-Percha-Material 105 automatisch durch das Dentalobjekt 100 auf die gewünschte Temperatur erwärmt, ohne dass hierzu weitere Vorrichtungen erforderlich sind. Beispielsweise wird das Gutta-Percha-Materials 105 hierbei auf eine Temperatur von 40°- 80° C erhöht.

Die mit dem Gutta-Percha-Material 105 präparierten Dentalobjekte 100 können beispielsweise mittels eines Heißluftstroms von 110 - 130 °C ca. 1 bis 2 min erwärmt werden. Die Oberflächentemperatur des Gutta-Percha-Materials 105 sollte 45 - 50 °C nicht überschreiten, um später am Patienten keine Verletzungen des Zahnfleisches oder Irritationen auf den Zähnen zu erzeugen. Das Dentalobjekt 100 wird anschließend händisch auf den Zahn gepresst und kann dann mit einem kühlen Luftstrom abgekühlt werden, so dass dieses seine Form behält. Das Gutta-Percha-Material 105 wird auch durch den Speichelfluss und den Blutfluss in den Zähnen automatisch innerhalb weniger Minuten abgekühlt.

Durch das Dentalobjekt 100 wird eine starke, einfach reversible Befestigung im Intraoralraum erreicht. Dabei ist besonders die Lösbarkeit und die Einfachheit der Befestigung durch die Patienten von Vorteil, damit diese eine gewohnte Mundhygiene durchführen können und keine Sekundärerkrankungen durch das Dentalobjekt 100 ausgelöst werden. Das Gutta-Percha-Material 105 dient als formgebendes Element und als Haftvermittler für die flexible Befestigung. Für besseren Verbund zum Gutta-Percha-Material 105 kann die zahninnenliegende Seite des Gehäuses 107 mittels Sandstrahlen aufgeraut werden und/oder eine Klebschicht 115 umfassen. Die Klebschicht 115 kann beispielsweise mit adhäsiven Klebeverfahren oder Sprühkleber erzeugt werden. Das Gehäuse 107 kann aber auch ein oder mehrere Rastelemente oder Verbindungselemente zum Herstellen eines Formschlusses zwischen dem Haftbereich und dem Sensor oder Sensorgehäuse umfassen. Das Verbindungselement kann beispielsweise durch eine oder mehrere Noppen gebildet sein.

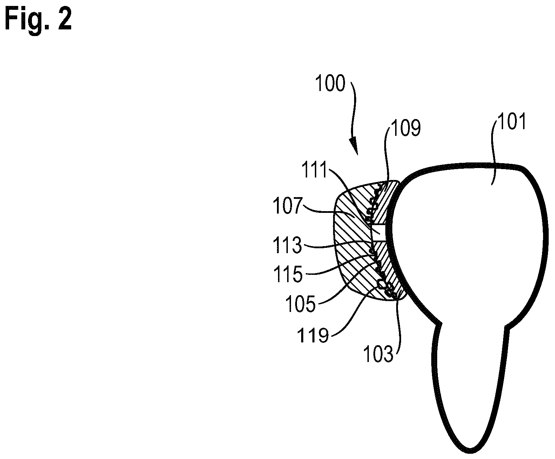

Fig. 2 zeigt eine weitere schematische Ansicht eines Dentalobjekts 100. Das Dentalobjekt 100 weist eine strukturierte Kontaktfläche 119 mit Vertiefungen oder Öffnungen auf, in denen sich das Gutta-Percha-Material 105 befindet. Durch die strukturierte Kontaktfläche 119 wird eine größere Oberfläche erreicht, so dass die Haftwirkung des Gutta-Percha-Materials 105 an dem Dentalobjekt 100 erhöht wird. Das Dentalobjekt 100 kann auch Rastelemente oder Verbindungselemente zum Befestigen des Gutta-Percha-Material 105 umfassen.

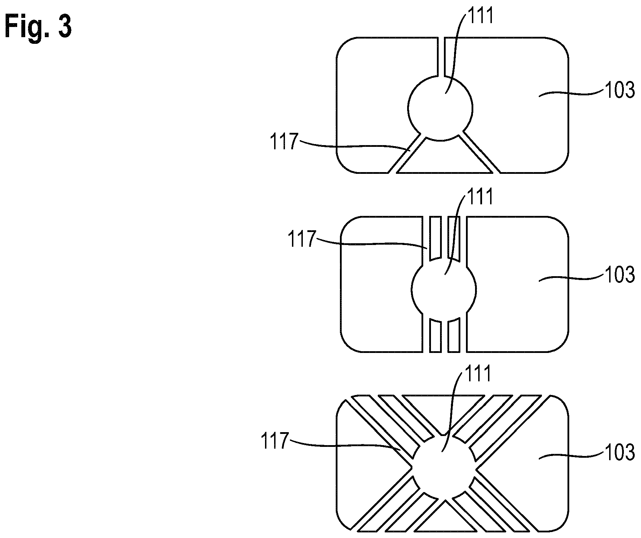

Fig. 3 zeigt eine schematische Ansicht des plastischen Haftbereichs 103 mit Kanälen 117 für einen Flüssigkeitsabfluss oder eine Luftzuführung. In dem Haftbereich 103 mit dem Gutta-Percha-Material 105 ist die Durchgangsöffnung 111 zum Ermöglichen einer Messung durch eine Sensoreinheit angeordnet. Die Durchgangsöffnung 111 bildet einen Messbereich für die Sensoreinheit.

Die Kanäle 117 sind in dem Haftbereich 103 durch Vertiefungen gebildet und dienen dazu Flüssigkeit (Speichel) zu der Sensoreinheit hinzuführen oder eine Ventilation des Messbereichs zu ermöglichen. Die Kanäle 117 können in horizontaler, diagonaler oder vertikaler Richtung angeordnet sein.

Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

Der Schutzbereich der vorliegenden Erfindung ist durch die Ansprüche gegeben und wird durch die in der Beschreibung erläuterten oder den Figuren gezeigten Merkmale nicht beschränkt.

### BEZUGSZEICHENLISTE

- 100: Dentalobjekt
- 101: Zahn
- 103: Haftbereich
- 105: Gutta-Percha-Material
- 107: Gehäuse
- 109: Schicht
- 111: Durchgangsöffnung
- 113: Einwölbung
- 115: Klebschicht
- 117: Kanal
- 119: Kontaktfläche

## Patentansprüche

1. Dentalobjekt (100) zum Befestigen an einem Zahn oder Zähnen (101) in einem Intraoralraum, mit:
- einem Haftbereich (103), der ein Gutta-Percha-Material (105) zum Befestigen des Dentalobjekts (100) an dem Zahn (101) umfasst,
wobei das Dentalobjekt (100) eine Sensorik, ein Elektronikbauteil, eine Batterie, ein Gehäuse (107) und/oder eine Halteplatte umfasst.

2. Dentalobjekt (100) nach Anspruch 1, wobei eine Schicht (109) des Gutta-Percha-Materials (105) an dem Gehäuse (107) befestigt ist.

3. Dentalobjekt (100) nach Anspruch 2, wobei die Schicht (109) des Gutta-Percha-Materials (105) mittels einer Klebeschicht (115) an dem Gehäuse (107) befestigt ist und/oder das Gehäuse (107) eine strukturierte Oberfläche zum Befestigen des Gutta-Percha-Materials (105) und/oder ein oder mehrere Rastelemente zum Befestigen des Gutta-Percha-Materials (105) und/oder ein oder mehrere Verbindungselemente zum Herstellen eines Formschlusses zwischen einem Haftbereich und einem Sensor oder Sensorgehäuse umfasst.

4. Dentalobjekt (100) nach Anspruch 2 oder 3, wobei die Schicht (109) des Gutta-Percha-Materials (105) eine Dicke von 1µm bis 5 mm aufweist.

5. Dentalobjekt (100) nach einem der Ansprüche 1 bis 4, wobei das Gutta-Percha-Material (105) eine vorgefertigte Durchgangsöffnung (111) zum Führen von Flüssigkeit vom Zahn in das Innere des Gehäuses (107) umfasst.

6. Dentalobjekt (100) nach einem der vorangehenden Ansprüche, wobei das Gutta-Percha-Material (105) antibakterielle Partikel oder Substanzen umfasst.

7. Dentalobjekt (100) nach einem der vorangehenden Ansprüche, wobei das Gutta-Percha-Material (105) einen Weichmacher umfasst.

8. Dentalobjekt (100) nach einem der vorangehenden Ansprüche, wobei das Gutta-Percha-Material (105), der Sensor und/oder das Gehäuse (115) ein oder mehrere Kanäle (125) zum Führen von Speichel zu einer Sensoreinheit umfasst.

9. Dentalobjekt (100) nach Anspruch 8, wobei die Kanäle (117) sich schräg oder vertikal durch das Gutta-Percha-Material (105), den Sensor und/oder das Gehäuse erstrecken.

10. Dentalobjekt (100) nach einem der vorangehenden Ansprüche, wobei das Gutta-Percha-Material (105) anatomisch vorgeformt ist.

## Claims

1. A dental object (100) for attachment to a tooth or teeth (101) in an intraoral space, comprising:
- an adhesive region (103) comprising a gutta-percha material (105) for attaching the dental object (100) to the tooth (101),
wherein the dental object (100) comprises sensor technology, an electronic component, a battery, a housing (107) and/or a retaining plate.

2. The dental object (100) as claimed in claim 1, wherein a layer (109) of the gutta-percha material (105) is attached to the housing (107).

3. The dental object (100) as claimed in claim 2, wherein the layer (109) of the gutta-percha material (105) is attached to the housing (107) by means of an adhesive layer (115) and/or the housing (107) comprises a textured surface for attaching the gutta-percha material (105) and/or one or more latching elements for attaching the gutta-percha material (105) and/or one or more connecting elements for establishing a form fit between an adhesive region and a sensor or sensor housing.

4. The dental object (100) as claimed in claim 2 or 3, wherein the layer (109) of the gutta-percha material (105) has a thickness of 1 µm to 5 mm.

5. The dental object (100) as claimed in any of claims 1 to 4, wherein the gutta-percha material (105) comprises a pre-fabricated passage opening (111) for guiding fluid from the tooth into an interior of the housing (107).

6. The dental object (100) as claimed in any of the preceding claims, wherein the gutta-percha material (105) comprises antibacterial particles or substances.

7. The dental object (100) as claimed in any of the preceding claims, wherein the gutta-percha material (105) comprises a plasticizer.

8. The dental object (100) as claimed in any of the preceding claims, wherein the gutta-percha material (105), the sensor and/or the housing (115) comprises one or more channels (125) for guiding saliva to a sensor unit.

9. The dental object (100) as claimed in claim 8, wherein the channels (117) extend obliquely or vertically through the gutta-percha material (105), the sensor and/or the housing.

10. The dental object (100) as claimed in any of the preceding claims, wherein the gutta-percha material (105) is anatomically preformed.

## Revendications

1. Objet dentaire (100) destiné à être fixé à une dent ou à des dents (101) dans un espace intraoral, comprenant :
- une zone d'adhésion (103) comprenant un matériau gutta-percha (105) pour fixer l'objet dentaire (100) à la dent (101),
où l'objet dentaire (100) comprend un capteur, un composant électronique, une batterie, un boîtier (107) et/ou une plaque de support.

2. Objet dentaire (100) selon la revendication 1, où une couche (109) du matériau gutta-percha (105) est fixée au boîtier (107).

3. Objet dentaire (100) selon la revendication 2, où la couche (109) de matériau gutta-percha (105) est fixée au boîtier (107) au moyen d'une couche adhésive (115) et/ou le boîtier (107) comprend une surface structurée pour fixer le matériau gutta-percha (105) et/ou un ou plusieurs éléments d'encliquetage pour fixer le matériau gutta-percha (105) et/ou un ou plusieurs éléments de liaison pour réaliser une liaison par complémentarité de forme entre une zone adhésive et un capteur ou un boîtier de capteur.

4. Objet dentaire (100) selon la revendication 2 ou 3, où la couche (109) de matériau gutta-percha (105) présente une épaisseur de 1 µm à 5 mm.

5. Objet dentaire (100) selon l'une des revendications 1 à 4, où le matériau gutta-percha (105) comprend un orifice de passage préformé (111) pour guider le liquide de la dent vers l'intérieur du boîtier (107).

6. Objet dentaire (100) selon l'une des revendications précédentes, où le matériau gutta-percha (105) comprend des particules ou substances antibactériennes.

7. Objet dentaire (100) selon l'une des revendications précédentes, où le matériau gutta-percha (105) comprend un plastifiant.

8. Objet dentaire (100) selon l'une des revendications précédentes, où le matériau gutta-percha (105), le capteur et/ou le boîtier (115) comprend un ou plusieurs canaux (125) pour guider la salive vers une unité de détection.

9. Objet dentaire (100) selon la revendication 8, où les canaux (117) s'étendent obliquement ou verticalement à travers le matériau de gutta-percha (105), le capteur et/ou le boîtier.

10. Objet dentaire (100) selon l'une des revendications précédentes, où le matériau gutta-percha (105) est préformé de manière anatomique.
